(19) Europäisches Patentamt
European Patent Office
Office européen des brevets

(11) Publication number: **0 263 905 B1**

(12) **EUROPEAN PATENT SPECIFICATION**

(45) Date of publication of patent specification: **15.04.92**

(51) Int. Cl.5: **C07D 249/20**

(21) Application number: **86309122.9**

(22) Date of filing: **21.11.86**

The file contains technical information submitted after the application was filed and not included in this specification

# (54) Method for preparing 2-phenylbenzotriazoles and 2-phenylbenzotriazole-N-oxides.

(30) Priority: **16.09.86 JP 218864/86**
**16.09.86 JP 218865/86**

(43) Date of publication of application:
**20.04.88 Bulletin 88/16**

(45) Publication of the grant of the patent:
**15.04.92 Bulletin 92/16**

(84) Designated Contracting States:
**CH DE FR GB IT LI**

(56) References cited:
**EP-A- 0 130 938**

**PATENT ABSTRACTS OF JAPAN, vol. 9, no. 28 (C-264)[1751], 6th February 1985**

**PATENT ABSTRACTS OF JAPAN, vol. 9, no. 21 (C-263)[1744], 29th January 1985**

**PATENT ABSTRACTS OF JAPAN, vol. 11, no. 24 (C-399)[2471], 23rd January 1987**

**PATENT ABSTRACTS OF JAPAN, vol. 7, no. 11 (C-145)[1156], 18th January 1983**

**PATENT ABSTRACTS OF JAPAN, vol. 10, no. 366 (C-390)[2423], 6th December 1986**

(73) Proprietor: **CHEMIPRO KASEI KAISHA, LTD.**
**No. 2-15, Gokodori 5-chome Chuo-ku**
**Kobe-shi Hyogo-ken(JP)**

(72) Inventor: **Seino, Shuichi**
**No. 3-6, Karibadai 5-chome**
**Nishi-ku Kobe-shi Hyogo-ken(JP)**
Inventor: **Fujino, Tomizo**
**No. 2620-128, Ohkubo Ohkubo-cho**
**Akashi-shi Hyogo-ken(JP)**

(74) Representative: **Perry, Robert Edward et al**
**GILL JENNINGS & EVERY 53-64 Chancery Lane**
**London WC2A 1HN(GB)**

**EP 0 263 905 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a method for preparing 2-phenylbenzotriazoles and 2-phenylbenzotriazole-N-oxides.

2-Phenylbenzotriazoles of formula I, wherein $R_1$ is H, Cl, $C_{1-4}$ alkyl, $C_{1-4}$ alkoxy, COOH or $SO_3H$; $R_2$ is H, CL, $C_{1-4}$ alkyl or $C_{1-4}$ alkoxy; $R_3$ is H, Cl, $C_{1-12}$ alkyl, $C_{1-4}$ alkoxy, phenyl, ($C_{1-8}$ alkyl)phenyl, phenoxy or phenyl($C_{1-4}$ alkyl); $R_4$ is H, Cl, OH or $C_{1-4}$ alkoxy; and $R_5$ is H, $C_{1-12}$ alkyl or phenyl($C_{1-4}$ alkyl), are known, and have utility as ultra-violet absorbers which can be added to, say, plastics, paints and oils.

2-Phenylbenzotriazole-N-oxides of formula II, wherein $R_1$, $R_2$, $R_3$, $R_4$ and $R_5$ are as defined above, are valuable intermediates in the preparation of the 2-phenylbenzotriazoles (I).

These known compounds have previously been produced by chemically or electrolytically reducing nitroazobenzenes of formula III, wherein $R_1$, $R_2$, $R_3$, $R_4$ and $R_5$ are as defined above. However, the known methods are not always satisfactory.

JP-A-37/5934 and US-A-3773751 disclose chemically reducing nitroazobenzenes (III) in an alcoholic sodium hyroxide solution with zinc powder. The yield is satisfactory but zinc sludge is produced, causing waste water contamination.

US-A-2362988 discloses chemical reduction of nitroazobenzenes (III) using ammonium sulphide, an alkali sulphide, zinc-ammonia, hydrogen sulphide-sodium or zinc-hydrochloric acid. This method produces a large amount of sulphite or zinc salts, thus contaminating waste water. Further, sulphites generates sulphurous acid gas, and the sulphide reducing agents generate poisonous hydrogen sulphide.

JP-A-51138679 and JP-A-51138680 disclose reduction using hydrogen under pressure. JP-A-5088072 discloses reduction using hydrazine. These methods give poor yields and are not economic. Further, it is impossible to obtain the desired product in high purity, owing to side-reactions. For example, when producing a chlorine-containing product, dechlorination occurs.

JP-A-59170172 and JP-A-51172481 disclose reduction with alcohols in the presence of quinones. However, quinones irritate the skin and mucous membrane, and can cause skin rash. Therefore, great care must be taken in handling the quinone catalyst, so that humans do not come into contact with the quinones, whether as liquid or as vapour. In addition to this disadvantage, the quinone catalyst changes in quality, and its catalytic effect is reduced, during use. Furthermore, recovery of the used catalyst is difficult.

EP-A-0130938 discloses a process for the preparation of 2-(2'-hydroxyphenyl)benztriazoles from 2-nitro-2'-hydroxyazobenzenes in a strongly basic medium in the presence of an aromatic catalyst such as benzoquinone or naphthoquinone, and using an alcohol as a reducing agent.

EP-A-0257151 and EP-A-0259530 (European Patent Applications Nos. 86306514.0 and 86307057.9) disclose improved processes, involving respectively reduction with a saccharide in the presence of a hydrogen transfer catalyst and base, and reduction with an aldehyde in the presence of an aromatic ketone and base. Alcohols are given as examples of inert solvents which can be used.

According to first and second aspects of the present invention, a method for preparing a 2-phenylbenzotriazole (I) as defined above comprises reducing a nitroazobenzene (III) as defined above or a 2-phenylbenzotriazole-N-oxide (II) as defined above, respectively, with a primary or secondary alcohol in the presence of base and an aromatic ketone selected from 9-fluorenone and benzanthrone.

According to a third aspect of the invention, a method for preparing a 2-phenylbenzotriazole-N-oxide (II) as defined above comprises reducing a nitroazobenzene (III) as defined above with a primary or secondary alcohol in the presence of base and an aromatic ketone selected from 9-fluorenone and benzanthrone.

The processes of the invention give technically and economically satisfactory results, without causing environmental pollution. The desired product is produced in higher purity, and has better thermostability, than has previously been disclosed.

The method according to the first aspect of the invention, i.e. III→I, can be carried out in either one or two steps, depending on the conditions such as temperature and the amount of the alcohol which is used. For a one-step reaction, a suitable temperature is 60 to 110°C, and a suitable period is 3 to 12 hours. Preferably, 0.9 to 1.3 mols of the primary alcohol or 1.8 to 2.6 mol of the secondary alcohol reducing agent are used per mol of the starting material (III).

The two-step method is sometimes advantageous in terms of product purity and yield, despite the use of two steps. In the first step, 0.4 to 0.7 mol primary alcohol or 0.8 to 1.4 mol secondary alcohol are preferably used per mol starting material (III); the temperature is suitably 50 or 60 to 100°C, e.g. for 2 to 10 hours. In the second step, 0.4 to 0.7 mol primary alcohol or 0.8 to 1.4 mol secondary alcohol are preferably used per mol of the resulting intermediate (II); the temperature is suitably 70 to 110°C, e.g. for 1 to 4 hours.

The given preferred temperatures are also preferred for the independent III→II and II→I methods according to the second and third aspects of the invention.

If a primary alcohol of the formula $RCH_2OH$, R being H or alkyl, is used, the novel reaction produces RCOOH and $H_2O$. If a secondary alcohol of the formula R'R''CHOH, R' and R'' being the same or different alkyl groups, is used R'COR'' and $H_2O$ are formed.

In order that the III→I, III→II or II→I process should proceed smoothly, it is preferably conducted in aqueous solution, or in an inert solvent such as an alcohol (e.g. excess of the alcohol reducing agent), toluene, acetone, dimethyl sulphoxide or acetonitrile; a mixture of the inert solvent with water may also be used. If necessary or desired, a surface-active agent or a phase-transfer catalyst may also be used.

Examples of nitroazobenzenes of formula III are given in European Patent Application No. 86306514.0; particular examples are:
2-nitro-4-chloro-2'-hydroxy-3'-t-butyl-5'-methylazobenzene,
2-nitro-2'-hydroxy-5'-methylazobenzene,
2-nitro-2'-hydroxy-5'-t-octylazobenzene,
2-nitro-2'-hydroxy-5'-t-butylazobenzene,
2-nitro-4-chloro-2'-hydroxy-3',5'-di-t-butylazobenzene,
2-nitro-2'-hydroxy-3',5'-di-t-amylazobenzene,
2-nitro-2'-hydroxy-3',5'-di-t-butylazobenzene,
2-nitro-2'-hydroxy-3'-t-butyl-5'-methylazobenzene,
2-nitro-2',4'-dihydroxyazobenzene,
2-nitro-4-chloro-2',4'-dihydroxyazobenzene,
2-nitro-2'-hydroxy-4'-methoxyazobenzene,
2-nitro-4-chloro-2'-hydroxy-3',5'-di-t-amylazobenzene,
2-nitro-2'-hydroxy-5'-t-amylazobenzene,
2-nitro-4'-chloro-2'-hydroxy-5'-t-amylazobenzene,
2-nitro-2'-hydroxy-3',5'-di(α,α-dimethylbenzyl)azobenzene,
2-nitro-4-chloro-2'-hydroxy-3',5'-di(α,α-dimethylbenzyl)azobenzene,
2-nitro-2'-hydroxy-3'-α-methylbenzyl-5'-methylazobenzene,
2-nitro-4-chloro-2'-hydroxy-3'-α-methylbenzyl-5'-methylazobenzene,
2-nitro-2'-hydroxy-5'-n-dodecylazobenzene,
2-nitro-4-chloro-2'-hydroxy-5'-n-dodecylazobenzene,
2-nitro-2'-hydroxy-3',5'-di-t-octylazobenzene,
2-nitro-4-chloro-2'-hydroxy-3',5'-di-t-octylazobenzene,
2-nitro-4-chloro-2'-hydroxy-5'-t-octylazobenzene,
2-nitro-4-methyl-2'-hydroxy-5'-methylazobenzene,
2-nitro-4-methyl-2'-hydroxy-3'-t-butyl-5-methylazobenzene,
2-nitro-4-n-butyl-2'-hydroxy-3',5'-di-t-butylazobenzene,
2-nitro-4-n-butyl-2'-hydroxy-3'-sec-butyl-5'-di-t-butylazobenzene,
2-nitro-4-t-butyl-2'-hydroxy-3'-sec-butyl-5'-di-t-butylazobenzene,
2-nitro-4,6-dichloro-2'-hydroxy-5'-t-butylazobenzene,
2-nitro-4,6-dichloro-2'-hydroxy-3,5'-di-t-butylazobenzene, and
2-nitro-4-carboxy-2'-hydroxy-5-methylazobenzene.

Examples of 2-phenylbenzotriazole-N-oxides of formula II are given in European Patent Application No. 86306514.0; those and others are:
2-(2-hydroxy-5-methylphenyl)benzotriazole-N-oxide,
2-(2-hydroxy-5-t-butylphenyl)benzotriazole-N-oxide,
2-(2-hydroxy-5-t-octylphenyl)benzotriazole-N-oxide,
2-(2,4-dihydroxyphenyl)-5-chlorobenzotraizole-N-oxide,
2-(2,4-dihydroxyphenyl)benzotriazole-N-oxide,
2-(2,4'-dihydroxyphenyl)-5-chlorobenzotriazole-N-oxide,
2-(2-hydroxy-4'-methoxyphenyl)benzotriazole-N-oxide,
2-(2-hydroxy-5-t-amylphenyl)benzotriazole-N-oxide,
2-(2-hydroxy-5-n-dodecylphenyl)benzotriazole-N-oxide,
2-(2-hydroxy-5-n-dodecylphenyl)-5-chlorobenzotriazole-N-oxide,
2-(2-hydroxy-5-t-octylphenyl)-5-chlorobenzotriazole-N-oxide,
2-(2-hydroxy-5-methylphenyl)-5-methylbenzotriazole-N-oxide,
2-(2-hydroxy-5-methylphenyl)-5-carboxybenzotriazole-N-oxide,
2-(2-hydroxy-3-t-butyl-5-methylphenyl)-5-chlorobenzotriazole-N-oxide,

2-(2-hydroxy-3,5-di-t-butylphenyl)-5-chlorobenzotriazole -N-oxide,
2-(2-hydroxy-3,5-di-t-amylphenyl)benzotriazole-N-oxide,
2-(2-hydroxy-3,5'-di-t-butylphenyl)benzotriazole-N-oxide,
2-(2-hydroxy-3-t-butyl-5-methylphenyl)benzotriazole-N-oxide,
2-(2-hydroxy-3,5'-di-t-octylphenyl)benzotriazole-N-oxide,
2-(2-hydroxy-3,5'-di-t-octylphenyl)-5-chlorobenzotriazole-N-oxide,
2-(2-hydroxy-3-t-butyl-5-methylphenyl)-5-methylbenzotriazole-N-oxide,
2-(2-hydroxy-3,5'-di-t-butylphenyl)-5-n-butylbenzotriazole-N-oxide,
2-(2-hydroxy-3-sec-butyl-5-t-butylphenyl)-5-n-butylbenzotriazole-N-oxide,
2-(2-hydroxy-3-sec-butyl-5-t-butylphenyl)-5-t-butylbenzotriazole-N-oxide,
2-(2-hydroxy-3,5-di-t-butylphenyl)-5,7-dichlorobenzotriazole-N-oxide
2-(2-hydroxy-3,5-di-t-amylphenyl)-5-chlorobenzotriazole-N-oxide,
2-[2-hydroxy-3,5-di-($\alpha$,$\alpha$-dimethylbenzyl)phenyl]benzotriazole-N-oxide,
2-[2-hydroxy-3,5-di-($\alpha$,$\alpha$-dimethylbenzyl)phenyl]-5-chlorobenzotriazole-N-oxide,
2-(2-hydroxy-$\alpha$-methylbenzyl-5-methylphenyl)benzotriazole-N-oxide, and
2-(2-hydroxy-$\alpha$-methylbenzyl-5-methylphenyl)chlorobenzotriazole-N-oxide.

The 2-phenylbenzotriazole-N-oxides (II) are preferably prepared by reduction of the nitroazobenzenes (III). Alternatively, for use in the II→I conversion according to the invention, they may be obtained by any other suitable process.

The nitroazobenzenes (III) may be obtained by a diazotisation reaction between a nitroaniline of formula IV and a phenol of formula V.

Examples of alcohols which can be used as reducing agents in accordance with the present invention are $C_{1-14}$ alkanols, including methanol, ethanol, 1-propanol, 2-propanol, 1-butanol, 2-butanol, 2-methyl-1-propanol, 1-pentanol, 2-pentanol, 3-methyl-1-butanol, 1-hexanol, 2-methylpentanol, 4-methylpentanol, 2-ethylbutanol, 1-heptanol, 2-heptanol, 3-heptanol, 1-octanol, 2-octanol, 2-ethylhexanol, 1-nonanol, 3,5,5-trimethylhexanol, 1-decanol, 1-undecanol, 1-dodecanol and 1-tetradecanol. 1-Propanol, 2-propanol, 1-butanol, 2-butanol, 1-pentanol, 2-pentanol and 1-octanol are preferred.

The alcohol reducing agent is preferably used in an amount greater than stoichiometric. Generally, alcohols are used also as a solvent, and they may then be used in an amount of 1 to 20, preferably 2 to 8 parts by weight per part by weight of the starting material (II or III).

Two or more alcohol reducing agents may be used in admixture, and such a combination sometimes provides a good result.

The alcohol may be used in aqueous solution; in such a case, the concentration of alcohol in the aqueous solution should preferably be at least 50% by weight. The fact that alcohols can be used in an aqueous solution brings the great advantage, for the purposes of industrial production, that the recovered alcohol can be re-used without being dehydrated.

The aromatic ketone is 9-fluorenone or benzanthrone. These two ketones may be used in admixture, and this is sometimes preferred.

The aromatic ketone is used generally in an amount of 0.2 to 30%, preferably 2 to 20%, by weight based on the weight of the starting material, i.e. nitroazobenzene (III) or 2-phenylbenzotriazole-N-oxide (II).

The given aromatic ketone catalysts are generally liquid and are generally soluble in organic solvents such as benzene, toluene, xylene or an alcohol. Accordingly, the used aromatic ketone catalyst can easily be recovered from the reaction system by extraction distillation. The recovered catalyst can be reused for another reaction. The possibility of continued use of the catalyst is an important advantage of the present invention.

The base used in the present invention is, for example, sodium hydroxide or potassium hydroxide. Such a base is preferably used in an amount of 1 to 12 mol, more preferably 2 to 8 mol, per mol of the starting material, i.e. nitroazobenzene (III) or 2-phenylbenzotriazole-N-oxide (II).

The present invention is further illustrated by the following Examples.

Example 1

2-nitro-2'-hydroxy-3',5'-di-t-amylazobenzene (25.5 g) was added to a mixture of n-butanol (80 g), water (14 g) and 97 % sodium hydroxide (8.2 g), and the resultant mixture was stirred while raising temperature to 65 °C. Thereafter, the mixture was cooled to 50 °C, and 9-fluorenone (2.0 g) was added to the mixture. The reaction mixture was then heated to 90 °C in one hour, and the mixture was stirred at 90~96 °C for four hours to effect reaction; almost all of the azobenzene disappeared. Thereafter, water (50 ml) was added to the reaction liquor, and the reaction liquor was neutralized to pH 8~9 with 62 % sulfuric acid (13 g) to

precipitate a crystal. The crystal thus obtained was filtered to separate the crystal, and the separated crystal was then fully washed with water and further with a small amount of methanol. The washed crystal was then dried, thus producing 23.2 g of 2-(2'-hydroxy-3',5'-di-t-amylphenyl)benzotriazole-N-oxide having a melting point of 111 to 113 ° C at the yield of 95.0 %.

Example 2

The same procedure as in Example 1 was repeated, except that 2-nitro-2'-hydroxy-3',5'-di-t-amylazobenzene (25.5 g) was replaced by 2-nitro-2'-hydroxy-5'-t-octylazobenzene (23.7 g), thus producing 20.3 g of 2-(2-hydroxy-5-t-octylphenyl)benzotriazole-N-oxide having a melting point of 106 to 110 ° C at the yield of 90.0 %.

Example 3

The same procedure as in Example 1 was repeated, except that n-butanol (80g) was replaced by n-propanol (80 g), and that 2-nitro-2'-hydroxy-3',5'-di-t-amylazobenzene (25.5 g) was replaced by 2-nitro-2'-hydroxy-5-methylazobenzene (17.1 g), thus producing 14.1 g of 2-(2-hydroxy-5-methylphenyl)benzotriazole-N-oxide having a melting point of 138 to 140 ° C at the yield of 88 %.

Example 4

The same procedure as in Example 1 was repeated, except that 9-fluorenone was replaced respectively by (a) benzanthrone (2.0 g) and (b) benzophenone (2.0 g), thus producing 2-(2-hydroxy-3,5-di-t-amylphenyl)benzotriazole-N-oxide having a melting point of 110 to 113 ° C at the yields respectively of (a) 20.8 g, 85 % and (b) 15.9 g, 65 %.

Example 5

2-nitro-4-chloro-2'-hydroxy-3'-t-butyl-5'-methylazobenzene (23.2 g) was added to a mixture of n-butanol (60 g), water (10.6 g) and 97 % sodium hydroxide (6.9 g), and the resultant mixure was stirred while raising temperature to 65 ° C. Thereafter, the mixture was cooled to 50 ° C, and 9-fluorenone (1.5 g) was added to the mixture. The reaction mixture was then heated to 90 ° C in one hour, and the mixture was stirred at 90~96 ° C for five hours to effect reaction; almost all of the azobenzene disappeared. Thereafter, water (50 ml) was added to the reaction liquor, and the reaction liquor was neutralized to pH 8~9 with 62 % sulfuric acid (10 g), to precipitate a crystal. The crystal thus obtained was filtered to separate the crystal, and the separated crystal was then fully washed with water and further with a small amount of methanol. The washed crystal was then dried, thus producing 20.6 g of 2-(2-hydroxy-3-t-butyl-5-methylphenyl)-5-chlorobenzotriazole-N-oxide having a melting point of 160 to 163 ° C at the yield of 93.0 %.

Example 6

2-nitro-4-chloro-2'-hydroxy-3',5'-d-t-butylazobenzene (26.0 g) and 9-fluorenone (2.0 g) were added to a mixture of isopropyl alcohol (80g), water (14 g) and 97 % sodium hydroxide (11.0 g), and the resultant mixture was stirred at the boiling point of 79 ° C for 8 hours to effect reaction; almost all of the azobenzene disappeared.

Thereafter, water (50 ml) was added to the reaction liquor, and the reaction liquor was neutralized to pH 8 ~9 with 62 % sulfuric acid (21 g). The neutralized reaction liquor was cooled to 20 ° C, to precipitate a crystal. The crystal thus obtained was filtered to separate the crystal, and the separated crystal was then fully washed with water and further with a small amount of isopropyl alcohol. The washed crystal was then dried, thus producing 21.9 g of 2-(2-hydroxy-3,5-di-t-butylphenyl)-5-chlorobenzotriazole-N-oxide having a melting point of 178 to 180 ° C at the yield of 88.0 %.

Example 7

2-nitro-2'-hydroxy-5'-t-butylazobenzene (19.9 g) was added to a mixture of secondary butanol (80 g), water (14 g) and 97 % sodium hydroxide (11.2 g), and the resultant mixure was stirred while raising temperature to 65 ° C. Thereafter, the mixture was cooled to 50 ° C, and 9-fluorenone (1.5 g) was added to the mixture. The reaction mixture was then stirred at 90~95 ° C for five hours to effect reaction; almost all of

the azobenzene disappeared. Thereafter, water (50 ml) was added to the reaction liquor, and the reaction liquor was neutralized to pH 8 with 62 % sulfuric acid (15 g). The neutralized reaction liquor was then cooled to 20° C or lower to precipitate a crystal. The crystal thus obtained was filtered to separate the crystal, and the separated crystal was then fully washed with water and further with a small amount of methanol. The washed crystal was then dried, thus producing 15.5 g of 2-(2-hydroxy-5-t-butylphenyl)-benzotriazole-N-oxide having a melting point of 95 to 98° C at the yield, of 82.0 %.

Example 8

The same procedure as in Example 7 was repeated, except that secondary butanol (80g) was replaced by n-octanol (50 g), and that 2-nitro-2'-hydroxy-5'-t-butyl azobenzene (19.9 g) was replaced by 2-nitro-2'-hydroxy-3', 5'-di-t-butylazobenzene (23.7 g), thus producing 18.7 g of 2-(2-hydroxy-3,5-di-t-butylphenyl)-benzotriazole-N-oxide having a melting point of 168 to 171° C at the yield of 83.0 %.

Example 9

2-nitro-2'-hydroxy-3',5'-di-t-amylazobenzene (25.5 g) was added to a mixture of n-butanol (80 g), water (14 g) and 97 % sodium hydroxide (8.2 g), and the resultant mixure was stirred while raising temperature to 65° C. Thereafter, the mixture was cooled to 50° C, and 9-fluorenone (2.0 g) was added to the mixture. The reaction mixture was then heated to 90° C in one hour, and the mixture was stirred at 90~96° C for four hours to effect reaction; almost all of the azobenzene disappeared, to produce 2-(2-hydroxy-3,5-di-t-amylphenyl)benzotriazole-N-oxide.

In order to further conduct the reaction,

the resultant reaction liquor was cooled to 60° C, and 97% sodium, hydroxide (8.2 g) and 9-fluorenone (1.0 g) were added to the reaction liquor. The reaction mixture was then heated to 90° C in one hour, and was further stirred at 90~97° C for two hours; the N-oxide disappeared.

Thereafter, water (80 ml) was added to the reaction liquor, and the reaction liquor was then neutralized to pH 8~9 with 62 % sulfuric acid (26.0 g). The neutralized reaction liquor was then allowed to stand at 60~70° C to form a butanol layer containing 2-(2-hydroxy-3,5-di-t-amylphenyl)benzotriazole as the upper layer and an aqueous layer as the lower layer. After removing the lower aqueous layer, the upper butanol layer was placed in a distillation apparatus, and most of butanol was recovered under normal pressure or a slightly reduced pressure. Thereafter, the system was subjected to a vacuum of 2~4 mmHg, to recover 2.9 g of a fraction of 120~140° C. The recovered fraction was 9-fluorenone used as a catalyst, and 9-fluorenone can be reused as a catalyst for the next process. After removing the catalyst, the remaining material was washed with methanol to produce the final product, i.e. 2-(2-hydroxy -3,5-di-t-amylphenyl)benzotriazole(20.0 g)having a melting point of 78~80° C, at the yield of 85.4 %.

Example 10

The same procedure as in Example 9 was repeated, except that 2-nitro-2'-hydroxy-3',5'-di-t-amylazobenzene (25.5 g) was replaced by 2-nitro-2'-hydroxy-5'-t-octylazobenzene (23.7 g). Thus, after recovering the catalyst, the remaining material was washed with methanol to produce 18.0 g of 2-(2-hydroxy-5-t-octylphenyl)benzotriazole having a melting point of 103 to 105° C at the yield of 83.7 %.

Example 11

The same procedure as in Example 9 was repeated, except that n-butanol (80 g) was replaced by n-propanol (80 g). The desired product could be obtained at the same yield as in Example 9. 9-Fluorenone used in this Example was recovered as in Examples 9 and 10.

Example 12

The same procedure as in Example 9 was repeated, except that 9-fluorenone was replaced by benzanthrone. 19.4 g of 2-(2-hydroxy-3,5-di-t-amylphenyl)benzotriazole was obtained (Yield: 83%, melting point: 77-80° C).

Example 13

2-Nitro-4-chloro-2'-hydroxy-3'-t-butyl-5'-methyl azobenzene (23.2 g) was added to a mixture of n-butanol (60 g), water (10.6 g) and 97% sodium hydroxide (6.9 g), and the resultant mixture was stirred while raising the temperature to 65° C. Thereafter, the mixture was cooled to 50° C, and 9-fluorenone (1.5 g) was added to the mixture. The reaction mixture was then heated to 90° C in one hour, and the mixture was stirred at 90-96° C for five hours to effect reaction; almost all of the azobenzene disappeared.

Thereafter, water (50 ml) was added to the reaction mixture, and the mixture was neutralised to pH 8-9 with 62% sulfuric acid (10 g). The neutralised liquor was cooled to 20° C, to precipitate a crystal. The crystal thus precipitated was separated by filtration, thus producing the wet product, 22.9 g of 2-(2-hydroxy-3-t-butyl-5-methylphenyl)-5-chlorobenzotriazole-N-oxide (dry product: 20.6 g, yield: 93.0%, melting point: 160-163° C).

The wet product thus prepared was added to a mixture of 90 % n-butanol (66.7 g), 97% sodium hydroxide (16.5 g), and 9-fluorenone (1.5 g). The resultant mixture was heated to 90° C in one hour, and was reacted at 90 to 97° C for 3 hours; the N-oxide disappeared.

After adding water (80 ml) to the above-prepared reaction liquor, the resultant reaction liquor was neutralized to pH 8~9 with 62 % sulfuric acid (28 g). The neutralized liquor was cooled to 20° C, to precipitate a crystal. The crystal thus obtained was separated by filtration, and the separated crystal was fully washed with water and further with a small amount of methanol. The washed crystal was then dried, thus producing a light yellow crystal (18.2 g) of 2-(2-hydroxy-3-t-butyl-5-methylphenyl)-5-chlorobenzotriazole having a melting point of 139 to 140° C at the yield of 87 % (on the basis of azobenzene).

After filtering the N-oxide and the final product, the used catalyst can be recovered by allowing the filtrate to stand; thus, the butanol layer separated. The butanol layer thus separated is placed in a distillation apparatus. After recovering butanol, a fraction of 120~140° C was taken out under vacuum of 2~4 mmHg ⟨266-531 Pa⟩, thus recovering 80 % or more of the used catalyst.

Example 14

2-nitro-4-chloro-2'-hydroxy-3',5'-di-t-butylazobenzene (26.0 g) and 9-fluorenone (2.0 g) were added to a mixture of isopropyl alcohol (80g), water (14 g) and 97 % sodium hydroxide (11.0 g), and the resultant mixture was stirred at the boiling point of 79° C for 8 hours to effect reaction; almost all of the azobenzene disappeared, to produce 2-(2-hydroxy-3,5-di-t-butylphenyl)-5-chlorobenzotriazole-N-oxide.

Thereafter, the reaction liquor was cooled to 60° C, and 97 % sodium hydroxide (8.2 g) and then 9-fluorenone (1.0 g) were added to the reaction liquor. The resultant reaction liquor was stirred at the boiling point (79~ 82° C) for 6 hours; the N-oxide disappeared.

Thereafter, water (80 ml) was added to the reaction liquor, and the reaction liquor was neutralized to pH 8 ~9 with 62 % sulfuric acid (36 g). The neutralized liquor was cooled to 20° C, to precipitate a crystal. The crystal thus precipitated was separated by filtration, and the separated crystal was washed with water and further with a small amount of isopropyl alcohol. The washed crystal was dried, thus producing 18.6 g of 2-(2-hydroxy-3,5-di-t-butylphenyl)-5-chlorobenzotriazole having a melting point of 153-155° C at the yield of 78 %.

Example 15

9-fluorenone (2.5 g) was added to a mixture of secondary butanol (80g), water (14 g) and 97 % sodium hydroxide (22.4 g), and 2-nitro-2'-hydroxy-5'-t-butylazobenzene (19.9 g) was added to the resultant mixture for one hour while stirring at 80° C. The mixture was further stirred at 90~97° C for 4 hours; almost all of the azobenzene disappeared, to produce 2-(2-hydroxy-5-t-butylphenyl)benzotriazole-N-oxide. The resultant reaction liquor was further stirred for 4 hours; the N-oxide disappeared.

After adding water (80 ml) to the resultant reaction liquor, the liquor was distilled to recover an excess amount of secondary butanol. Toluene (50 ml) was then added to the remaining liquor, and the liquor was neutralized to pH 8 with 62 % sulfuric acid (31 g). The neutralized liquor was allowed to stand at 70° C, and the liquor was separated into the toluene layer containing the desired product as the upper layer and an aqueous layer containing sodium sulfate as the lower layer. The upper toluene layer was placed in a distillation apparatus, and most of toluene was removed by distillation under normal pressure. Thereafter, a fraction (2.5 g) at 120~140° C was recovered under a vacuum of 2~4 mmHg ⟨266-531 Pa⟩. The fraction thus obtained was the used catalyst, 9-fluorenone, and the catalyst thus recovered can be reused for the next process. After recovering the catalyst, the desired product, i.e. 13.5g of 2-(2-hydroxy-5-t-butylphenyl)-benzotriazole having a melting point of 96~98° C, was obtained at the yield of 76 %.

Example 16

2-nitro-2'-hydroxy-3',5'-di-t-butylazobenzene (23.7 g) was added to a mixture of n-octanol (50 g), water (14 g) and 97 % sodium hydroxide (8.2 g), and the resultant mixture was heated to 65 ° C. The mixture was then cooled to 50 ° C, and 9-fluorenone (2.0 g) was added thereto. The resultant mixture was stirred at 90~97 ° C for 2 hours; almost all of the azobenzene disappeared, to produce 2-(2-hydroxy-3,5-di-t-butylphenyl)benzotriazole-N-oxide.

Thereafter, the reaction liquor was cooled to 60 ° C, and 97 % sodium hydroxide (8.2 g) and then 9-fluorenone (1.0 g) were added to the reaction liquor. The resultant reaction liquor was raised to 90 ° C and stirred at 90~ 97 ° C for 2 hours; the N-oxide disappeared.

Thereafter, water (80 ml) was added to the reaction liquor, and the reaction liquor was neutralized to pH 8~9 with 62 % sulfuric acid (26 g). The neutralized liquor was cooled to 20 ° C, to precipitate a crystal. The crystal thus precipitated was separated by filtration, and the separated crystal was fully washed with water and further with a small amount of methanol. The washed crystal was dried, thus producing 17.0 g of 2-(2-hydroxy -3,5-di-t-butylphenyl)benzotriazole having a melting point of 150~152 ° C at the yield of 79 %.

Example 17

2-nitro-2'-hydroxy-5'-t-octylazobenzene (23.7 g) was added to a mixture of n-butanol (60g), water (12 g) and 97 % sodium hydroxide (16.5 g), and the resultant mixture was heated to 50 ° C while stirring. 9-fluorenone (2.0 g) was added to the mixture, and the resultant mixture was heated to 80 ° C in two hours. The mixture was then stirred at 80~84 ° C for 3.5 hours; thus, the reduction reaction completed.

After adding water (80 ml) to the resultant reaction liquor, the liquor was neutralized to pH 8 with 62 % sulfuric acid (28 g). The neutralized liquor was allowed to stand at 60~70 ° C, and the liquor was separated into the butanol layer containing the desired product, i.e. 2-(2-hydroxy-5-t-octylphenyl)benzotriazole, as the upper layer, and an aqueous layer as the lower layer. After removing the lower aqueous layer, the upper butanol layer was placed in a distillation apparatus, and most of butanol was recovered by distillation under normal pressure or slightly reduced pressure. Thereafter, a fraction 1.8 g at 120~140 ° C was recovered under vacuum of 2~3 mmHg ⟨266-399 Pa⟩. The fraction thus obtained was the used catalyst, i.e. 9-fluorenone, and the catalyst thus recovered can be reused for the next step. After recovering the catalyst, the remaining material was washed with methanol to produce the desired product, i.e. 17.4 g of 2-(2-hydroxy-5-t-octylphenyl)benzotriazole having a melting point of 103~105 ° C, at the yield of 81.0 %.

Example 18

2-(2-hydroxy-3,5-di-t-amylphenyl)benzotriazole-N-oxide (23.2 g) was added to a mixture of n-butanol (60g), water (12 g) and 97 % sodium hydroxide (16.5 g), and 9-fluorenone (2.0 g) was further added to the resultant mixture. The resultant mixture was heated to 80 ° C, and was further stirred at 80~85 ° C for 2.5 hours; thus, the reduction reaction completed. The reaction liquor was treated in the same manner as in Example 17 to recover butanol, and 1.8 g of 9-fluorenone catalyst was recovered as a fraction at 120~130 ° C under vacuum of 2 ~3 mmHg ⟨266-399 Pa⟩. After recovering the catalyst, the remaining material was washed with methanol to produce the desired product,i.e. 20.5 g of 2-(2-hydroxy-3,5-di-t-amylphenyl) benzotriazole having a melting point of 79~80 ° C, at the yield of 92.8 %.

## Claims

**1.** A method for preparing a 2-phenylbenzotriazole of formula I

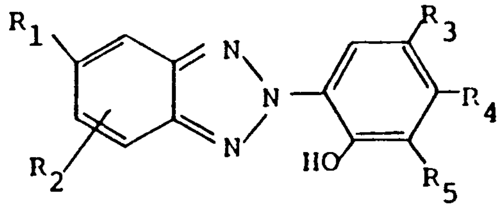

(I)

wherein $R_1$ is H, Cl, $C_{1-4}$ alkyl, $C_{1-4}$ alkoxy, COOH or $SO_3H$; $R_2$ is H, Cl, $C_{1-4}$ alkyl or $C_{1-4}$ alkoxy; $R_3$ is H, Cl, $C_{1-12}$ alkyl, $C_{1-4}$ alkoxy, phenyl, ($C_{1-8}$ alkyl)phenyl, phenoxy or phenyl($C_{1-4}$ alkyl); $R_4$ is H, Cl, OH or $C_{1-4}$ alkoxy; and $R_5$ is H, $C_{1-12}$ alkyl or phenyl($C_{1-4}$ alkyl), which comprises reducing a nitroazobenzene of formula III

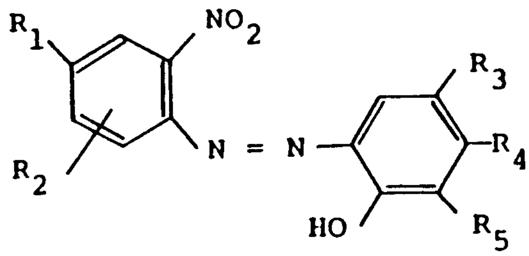

(III)

wherein $R_1$, $R_2$, $R_3$, $R_4$ and $R_5$ are as defined above, with a primary or secondary alcohol, in the presence of base and an aromatic ketone selected from 9-fluorenone and benzanthrone.

**2.** A method according to claim 1, wherein 0.9 to 1.3 mol primary alcohol or 1.8 to 2.6 mol secondary alcohol are used per mol nitroazobenzene (III).

**3.** A method according to claim 1, wherein, in the first of two steps, 0.4 to 0.7 mol primary alcohol or 0.8 to 1.4 mol secondary alcohol are used per mol nitroazobenzene (III), to give an intermediate which is a 2-phenylbenzotriazole-N-oxide of formula II

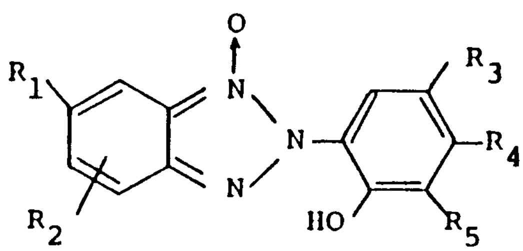

(II)

wherein $R_1$, $R_2$, $R_3$, $R_4$ and $R_5$ are as defined in claim 1, and, in the second step, 0.4 to 0.7 mol primary alcohol or 0.8 to 1.4 mol secondary alcohol are used per mol intermediate (II).

**4.** A method for preparing a 2-phenylbenzotriazole (I) as defined in claim 1, which comprises reducing a 2-phenylbenzotriazole-N-oxide (II) as defined in claim 3 with, per mol thereof, 0.4 to 0.7 mol primary alcohol or 0.8 to 1.4 mol secondary alcohol, in the presence of base and an aromatic ketone as defined in claim 1.

**5.** A method for preparing a 2-phenylbenzotriazole-N-oxide (II) as defined in claim 3, which comprises reducing nitroazobenzene (III) as defined in claim 1 with, per mol thereof, 0.4 to 0.7 mol primary alcohol or 0.8 to 1.4 mol secondary alcohol, in the presence of base and an aromatic ketone as defined in claim 1.

**6.** A method according to any preceding claim, wherein the alcohol reducing agent is a $C_{1-14}$ alkanol.

**7.** A method according to any preceding claim, wherein the aromatic ketone is used in an amount of 0.2 to 30% by weight, based on the weight of the starting material (II or III).

**8.** A method according to any preceding claim, wherein the base is sodium hydroxide or potassium hydroxide in an amount of 1 to 12 mol per mol starting material (II or III).

## Revendications

**1.** Procédé pour la préparation d'un 2-phénylbenzotriazole de formule I

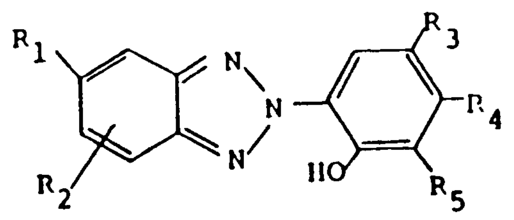

(I)

dans laquelle $R_1$ est H, Cl, un groupe alkyle en $C_{1-4}$, alcoxy en $C_{1-4}$, COOH ou $SO_3H$; $R_2$ est H, Cl, un groupe alkyle en $C_{1-4}$ ou alcoxy en $C_{1-4}$; $R_3$ est H, Cl, un groupe alkyle en $C_{1-12}$, alcoxy en $C_{1-4}$, phényle, alkyl($C_{1-8}$)-phényle, phénoxy ou phényl-alkyle($C_{1-4}$); $R_4$ est H, Cl, OH ou un groupe alcoxy en $C_{1-4}$; et $R_5$ est H, un groupe alkyle en $C_{1-12}$ ou phénylalkyle($C_{1-4}$), comprenant la réduction d'un nitroazobenzène de formule III

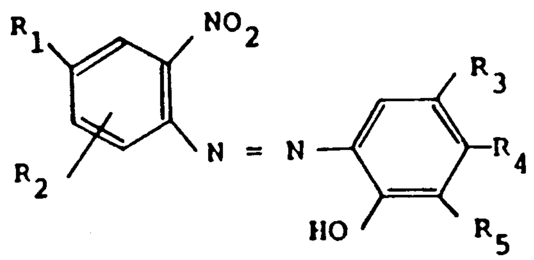

(III)

dans laquelle $R_1$, $R_2$, $R_3$, $R_4$ et $R_5$ sont tels que définis ci-dessus, à l'aide d'un alcool primaire ou secondaire, en présence d'une base et d'une cétone aromatique choisie parmi la 9-fluorénone et la benzanthrone.

**2.** Procédé selon la revendication 1, dans lequel on utilise de 0,9 à 1,3 mole d'alcool primaire ou de 1,8 à 2,6 moles d'alcool secondaire par mole de nitroazobenzène (III).

**3.** Procédé selon la revendication 1, dans lequel, dans la première de deux étapes, on utilise de 0,4 à 0,7 mole d'alcool primaire ou de 0,8 à 1,4 mole d'alcool secondaire par mole de nitroazobenzène (III), pour obtenir un produit intermédiaire qui est un N-oxyde de 2-phénylbenzotriazole de formule II

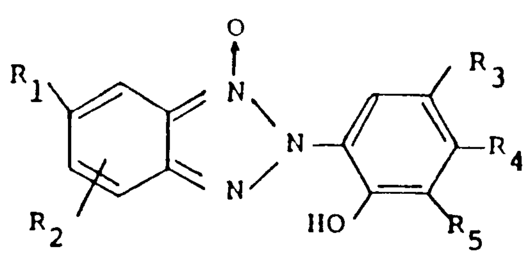

(II)

dans laquelle $R_1$, $R_2$, $R_3$, $R_4$ et $R_5$ sont tels que définis dans la revendication 1, et, dans la seconde étape, on utilise de 0,4 à 0,7 mole d'alcool primaire ou de 0,8 à 1,4 mole d'alcool secondaire par mole de produit intermédiaire (II).

**4.** Procédé pour la préparation d'un 2-phénylbenzotriazole (I) tel que défini dans la revendication 1, comprenant la réduction d'un N-oxyde de 2-phénylbenzotriazole (II), tel que défini dans la revendication 3, à l'aide, par mole de celui-ci, de 0,4 à 0,7 mole d'alcool primaire ou de 0,8 à 1,4 mole d'alcool secondaire, en présence d'une base et d'une cétone aromatique telle que définie dans la revendication

1.

**5.** Procédé pour la préparation d'un N-oxyde de 2-phénylbenzotriazole (II) tel que défini dans la revendication 3, comprenant la réduction d'un nitroazobenzène (III) tel que défini dans la revendication 1, à l'aide, par mole de celui-ci, de 0,4 à 0,7 mole d'alcool primaire ou de 0,8 à 1,4 mole d'alcool secondaire, en présence d'une base et d'une cétone aromatique telle que définie dans la revendication 1.

**6.** Procédé selon l'une quelconque des revendications précédentes, dans lequel le réducteur de type alcool est un alcanol en $C_{1-14}$.

**7.** Procédé selon l'une quelconque des revendications précédentes, dans lequel on utilise la cétone aromatique en une quantité allant de 0,2 à 30 % en poids, par rapport au poids du produit de départ (II) ou (III).

**8.** Procédé selon l'une quelconque des revendications précédentes, dans lequel la base est l'hydroxyde de sodium ou l'hydroxyde de potassium en une quantité de 1 à 12 moles par mole du produit de départ (II) ou (III).

## Patentansprüche

**1.** Verfahren zum Herstellen eines 2-Phenylbenzotriazols der Formel I

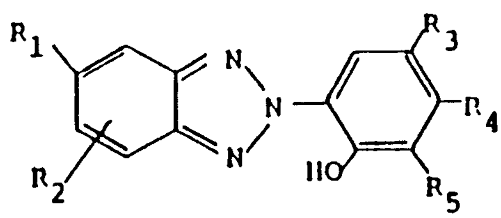

(I),

wobei $R_1$ die Bedeutung H, Cl, $C_{1-4}$-Alkyl, $C_{1-4}$-Alkoxy, COOH oder $SO_3H$ hat; $R_2$ die Bedeutung H, Cl, $C_{1-4}$-Alkyl oder $C_{1-4}$-Alkoxy hat; $R_3$ die Bedeutung H, Cl, $C_{1-12}$-Alkyl, $C_{1-4}$-Alkoxy, Phenyl, ($C_{1-8}$-Alkyl)phenyl, Phenoxy oder Phenyl($C_{1-4}$-alkyl) hat; $R_4$ die Bedeutung H, Cl, OH oder $C_{1-4}$-Alkoxy hat; und $R_5$ die Bedeutung H, $C_{1-12}$-Alkyl oder Phenyl($C_{1-4}$-alkyl) hat, wobei man ein Nitroazobenzol der Formel III

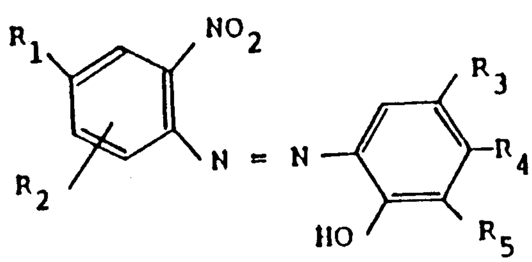

(III),

wobei $R_1$, $R_2$, $R_3$, $R_4$ und $R_5$ die vorstehend definierten Bedeutungen haben, mit einem primären oder sekundären Alkohol in Gegenwart von Base und einem aromatischen Keton reduziert, welches ausgewählt wird aus 9-Fluorenon und Benzanthron.

**2.** Verfahren nach Anspruch 1, wobei man pro Mol Nitroazobenzol(III) 0,9 bis 1,3 Mol primären Alkohol oder 1,8 bis 2,6 Mol sekundären Alkohol verwendet.

3. Verfahren nach Anspruch 1, wobei man im ersten von zwei Schritten 0,4 bis 0,7 Mol primären Alkohol oder 0,8 bis 1,4 Mol sekundären Alkohol pro Mol Nitroazobenzol (III) verwendet, um ein Zwischenprodukt in Gestalt eines 2-Phenylbenzotriazol-N-oxidsder Formel II

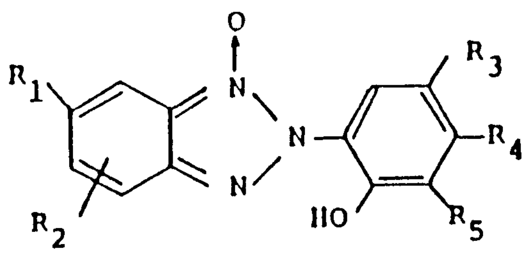

(II),

zu erhalten, wobei $R_1$, $R_2$, $R_3$, $R_4$ und $R_5$ die vorstehend in Anspruch 1 definierte Bedeutung haben und im zweiten Schritt 0,4 bis 0,7 Mol primären Alkohol oder 0,8 bis 1,4 sekundären Alkohol pro Mol Zwischenprodukt (II) verwendet.

4. Verfahren zum Herstellen eines Phenylbenzotriazols (I) wie in Anspruch 1 definiert, wobei man ein wie in Anspruch 3 definiertes 2-Phenylbenzotriazol-N-oxid (II) in Anwesenheit von Base und eines wie in Anspruch 1 definierten aromatischen Ketons mit 0,4 bis 0,7 Mol primärem Alkohol oder 0,8 bis 1,4 Mol sekundärem Alkohol pro Mol 2-Phenylbenzotriazol-N-oxid (II) reduziert.

5. Verfahren zum Herstellen eines Phenylbenzotriazol-N-oxids (II) wie in Anspruch 3 definiert, wobei man wie in Anspruch 1 definiertes Nitroazobenzol (III) in Gegenwart von Base und eines wie in Anspruch 1 definierten aromatischen Ketons mit 0,4 bis 0,7 Mol primärem Alkohol oder 0,8 bis 1,4 Mol sekundärem Alkohol pro Mol Nitroazobenzol (III) reduziert.

6. Verfahren nach einem der vorhergehenden Ansprüche, wobei man das Alkohol-Reduktionsmittel ein $C_{1-14}$-Alkanol ist.

7. Verfahren nach einem der vorhergehenden Ansprüche, wobei man das aromatische Keton, bezogen auf das Gewicht des Ausgangsstoffs (II oder III), in einer Menge von 0,2 bis 30 Gew.-% einsetzt.

8. Verfahren nach einem der vorhergehenden Ansprüche, wobei die Base Natriumhydroxid oder Kaliumhydroxid in einer Menge von 1 bis 12 Mol pro Mol Ausgangsstoff (II oder III) ist.